(19) Europäisches Patentamt
European Patent Office
Office européen des brevets

(11) Publication number: 0 154 417
A2

(12) # EUROPEAN PATENT APPLICATION

(21) Application number: 85300823.3

(22) Date of filing: 07.02.85

(51) Int. Cl.[4]: C 07 D 501/18

(30) Priority: 10.02.84 JP 24110/84

(43) Date of publication of application: 11.09.85 Bulletin 85/37

(84) Designated Contracting States: DE FR GB IT

(71) Applicant: YAMANOUCHI PHARMACEUTICAL CO. LTD.
No. 5-1 Nihonbashi-Honcho, 2-chome Chuo-ku
Tokyo(JP)

(72) Inventor: Shibanuma, Tadao
No. 596-11, Ohyaguchi
Urawa-shi saitama(JP)

(72) Inventor: Kanano, Kohji
1086-1, Ohaza Sanagaya Shiraoka-cho
Minamisaitama-gun Saitama(JP)

(72) Inventor: Nagano, Noriaki
24-13, Asamadai 3-chome
Ageo-shi Saitama(JP)

(72) Inventor: Murakami, Yukiyasu
306-3, Ohmaki
Urawa-shi Saitama(JP)

(72) Inventor: Hara, Ryuichiro
19-11, Hon-cho 3-chome
Nakano-ku Tokyo(JP)

(72) Inventor: Koda, Akio
5-3, Hibarigaoka Kita 4-chome
Hohya-shi Tokyo(JP)

(72) Inventor: Yamazaki, Atsuki
7-8-506, Shimura 2-chome
Itabashi-ku Tokyo(JP)

(74) Representative: Geering, Keith Edwin et al,
REDDIE & GROSE 16 Theobalds Road
London WC1X 8PL(GB)

(54) **7-Amino-3-substituted-methyl-3-cephem-4-carboxylic acid and lower alkylsilyl derivatives thereof, and their production.**

(57) Various 7-amino-3-substituted-methyl-3-cephem-4-carboxylic acids and lower alkylsilyl derivatives such as 7-amino-3-(2,3-cyclopenteno-1-pyridiniomethyl)-3-cephem-4-carboxylic acid iodide and trimethylsilyl 7-trimethylsilylamino-3-(5,6,7,8-tetrahydro-2-isoquinoliniomethyl)-3-cephem-4-carboxylate iodide are valuable intermediates in the preparation of cephalosporin antibacterial agents. A new process for the preparation of these acids and derivatives has been devised.

EP 0 154 417 A2

"7-AMINO-3-SUBSTITUTED-METHYL-3-CEPHEM-4-CARBOXYLIC ACID AND LOWER ALKYLSILYL DERIVATIVES THEREOF, AND THEIR PRODUCTION".

This invention relates to the production of chemical intermediates for the preparation of therapeutic cephalosporins, and to novel such intermediates per se. It meets a need for more efficient production of key intermediates for the preparation of antibacterial cephalosporins, e.g. such as those disclosed in EP Patent Application No.84306967.5.

According to the invention, compounds having the formula

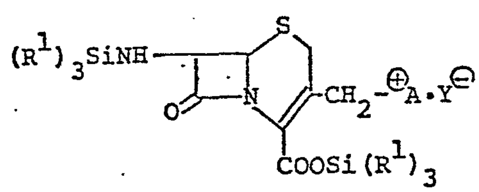

$(I_1)$

wherein $-^{\oplus}A$ represents

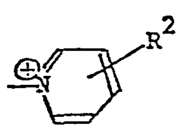 ,

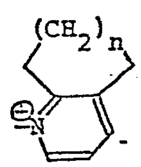

or

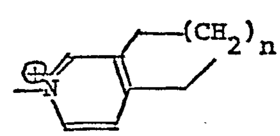

(wherein $R^2$ is a hydrogen atom or an amino group, and n is 1 or 2); $Y^{\ominus}$ represents an anion; and $R^1$ represents a lower alkyl group; are prepared by a process which comprises reacting 7-amino-3-halogenomethyl-3-cephem-4-carboxylic acid (II) or its salt.

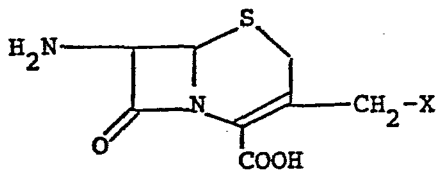 (II)

(wherein X is a halogen atom)

with (tri-loweralkylsilyl)trifluoroacetamide selected from

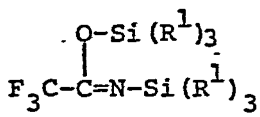 and 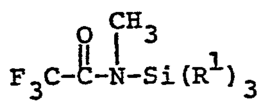

($III_1$) ($III_2$)

(wherein $R^1$ is as defined above)

and pyridine compound (IV) selected from

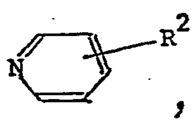, 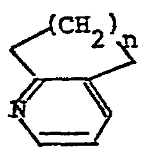 and 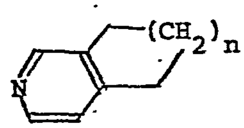

(wherein $R^2$ and n are as defined above).

If necessary, by releasing the protective groups [namely, $(R^1)_3Si-$], 7-amino-3-substituted-methyl-3-cephem-4-carboxylic acids (or carboxylates) of the formula ($I_2$)

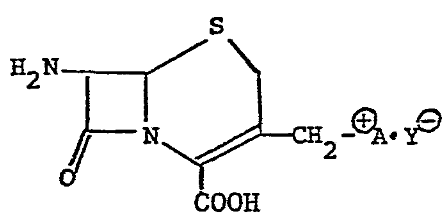 or 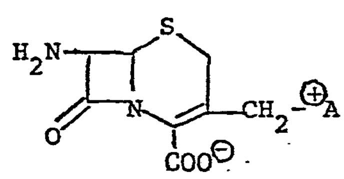

($I_2$)

are prepared.

Schematically, the reaction sequence may be illustrated as below.

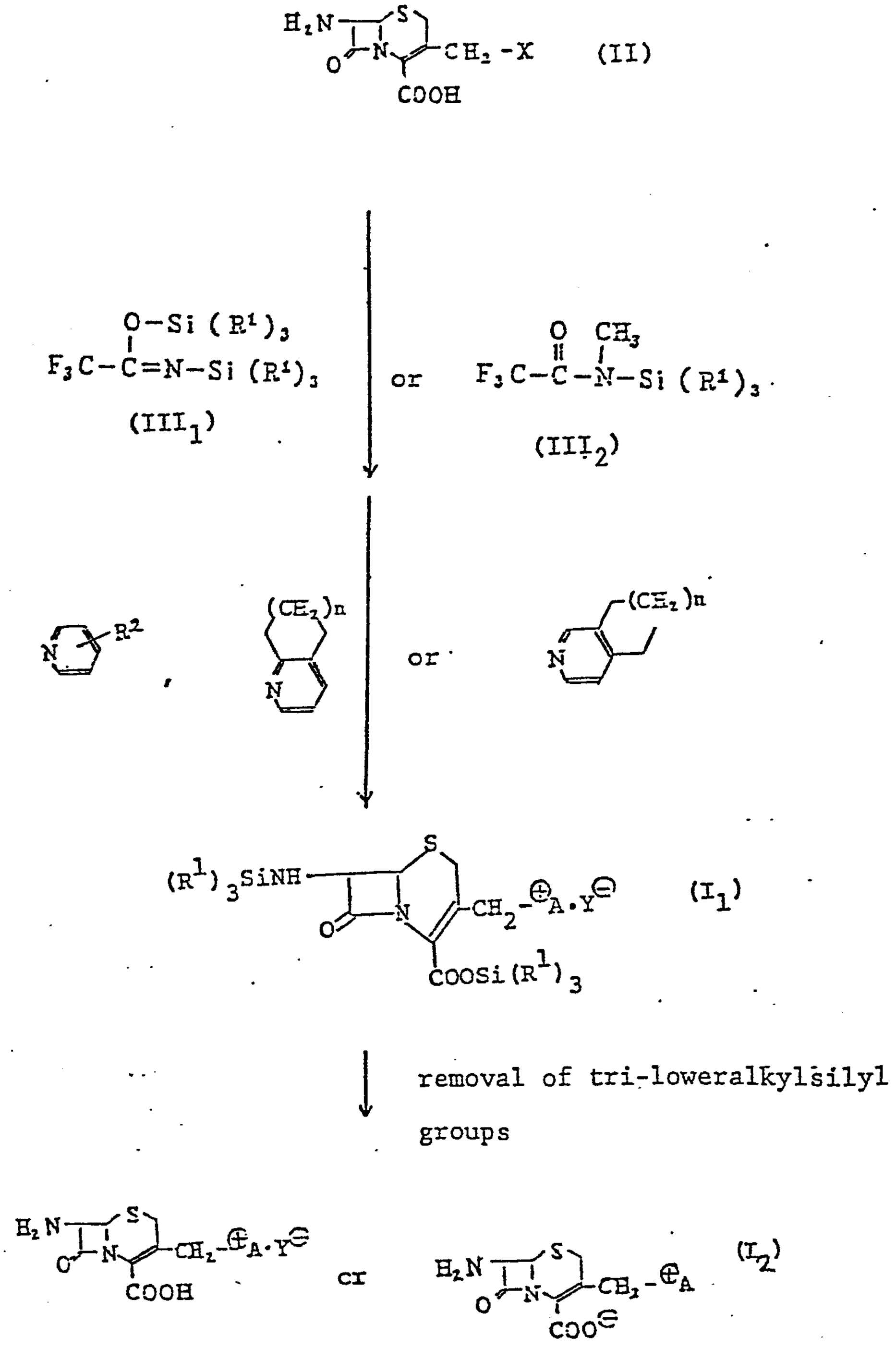


(II)

(III$_1$) or (III$_2$)

or

(I$_1$)

or (I$_2$)

Examples of the pyridine compounds used for the reaction of the present invention are pyridine, 4-aminopyridine, 3-aminopyridine, 2-aminopyridine, 2,3-cyclopentenopyridine, 5,6,7,8-tetrahydroquinoline, 3,4-cyclopentenopyridine, 5,6,7,8-tetrahydroisoquinoline, etc.

Examples of the group represented by $-^{\ominus}A$ are pyridinium, aminopyridinium ( 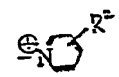 ), 2,3-cyclopenteno-1-pyridinium ( 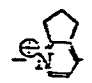 ), 5,6,7,8-tetrahydro-1-quinolinium ( 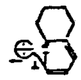 ), 3,4-cyclopenteno-1-pyridinium ( 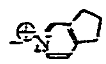 ), 5,6,7,8-tetrahydro-2-isoquinolinium ( 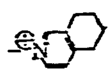 ), etc.

Examples of $Y^{\ominus}$ are inorganic anions such as halogen (e.g, $Cl^{\ominus}$, $I^{\ominus}$), and sulfonate (e.g. $HSO_4^{\ominus}$, $SO_4^{\ominus\ominus}$) anions;and organic anions such as benzenesulfonate ($C_6H_5SO_3^{\ominus}$), acetate ($CH_3COO^{\ominus}$), fumarate ($HOOC-CH=CH-COO^{\ominus}$), and citrate anions ($HOOC-CH_2C(OH)(COOH)CH_2-COO^{\ominus}$), etc.

Examples of "Halogen" in the foregoing definition are chlorine, bromine, iodine and fluorine.

Examples of "lower alkyl" are methyl, ethyl, propyl, butyl, iso-butyl, etc. Thus typical lower alkyl is a straight or branched carbon chain alkyl having 1 to 5 carbon atoms.

The compounds of formula ($I_1$) are novel compounds; and the compounds of formula ($I_2$) - except 7-amino-3-pyridiniomethyl-3-cephem-4-carboxylate, namely,

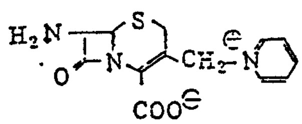

- are also novel compounds.

A known process for producing the above known compound (namely, 7-amino-3-pyridiniomethyl-3-cephem-4-carboxylate) is disclosed in unexamined Japanese patent application laid-open under the laying-open No. Sho.56-12397, and comprises hydrolysing cefaloridin which is a known valuable antibacterial compound. However, this known process is not economical, and is not suitable for the practical production of the compound.

The process of the present invention is entirely different from the known process, and has the characteristic that the readily available 7-amino-3-halogenomethyl cephalosporanic acid or salt is used as starting material. Further, the lower alkylsilyl compounds of this invention are easily soluble in organic solvents, and side reactions such as rearrangement of the double bond at the 2-position of the cephalosporin nucleus ($\Delta^3 \longrightarrow \Delta^2$ rearrangement) do not occur in the process of this invention even with nucleophilic pyridine compounds. Thus, the process of this invention can give product of good purity in good yield, and is suitable for the industrial manufacture of cephalosporin compounds.

In the process of this invention, 7-amino-3-halogenomethyl-3-cephem-4-carboxylic acid (II) or its salt can be reacted with (tri-loweralkylsilyl)trifluoroacetamide (III,

$III_2$) and pyridine compound (IV) in an organic solvent which is inert for the reaction.

The said trifluoroacetamide reactant is selected from O,N-bis(tri-loweralkylsilyl)trifluoroacetamide ($III_1$) and N-methyl-N-tri-loweralkylsilyltrifluoroacetamide ($III_2$). Preferred compounds ($III_1$) and ($III_2$) are e.g. O,N-bis-(trimethylsilyl)trifluoroacetamide

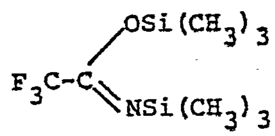

and N-methyl-N-trimethylsilyltrifluoroacetamide

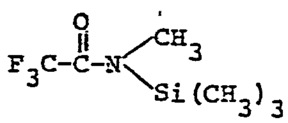

A two-step reaction is preferred, in which the starting acid or salt is first reacted with (tri-loweralkylsilyl)-trifluoroacetamide ($III_1$, $III_2$) followed by reaction with pyridine compound (IV), but all of the reactants can be reacted simultaneously.

The reaction proceeds easily at room temperature. When performing the reaction in 2 steps, the 1st step may be performed at room temperature and the 2nd step under heating or cooling at a temperature from -20°C to 40°C. Examples of the inert organic solvent usually used are dichloromethane,acetone, acetonitrile, tetra-hydrofuran, chloroform.

The compounds ($I_1$) can be used directly as starting materials for various cephalosporin compounds which may be valuable antibacterial agents; or, by releasing the tri-loweralkylsilyl protective groups, they may first be converted to compounds ($I_2$) which can then be used for the same purpose.

The removal of the protective groups is easily performed, e.g. by treatment with water or alcohol-solvent. Examples of alcohol are mono-OH alcohols (such as methanol, ethanol, propanol) and di-OH alcohols (such as 1,2-ethanediol, 1,3-propanediol, 1,3-butanediol). It is preferred to use 1,3-butanediol, in view of the easiness of the separation and purification of the formed compounds.

Cephalospórin derivatives of European Patent Application No.84 306967.5 can be prepared by amidation of the 7-amino group in the compounds of this invention using suitable acylating agents. This amidation reaction is explained below.

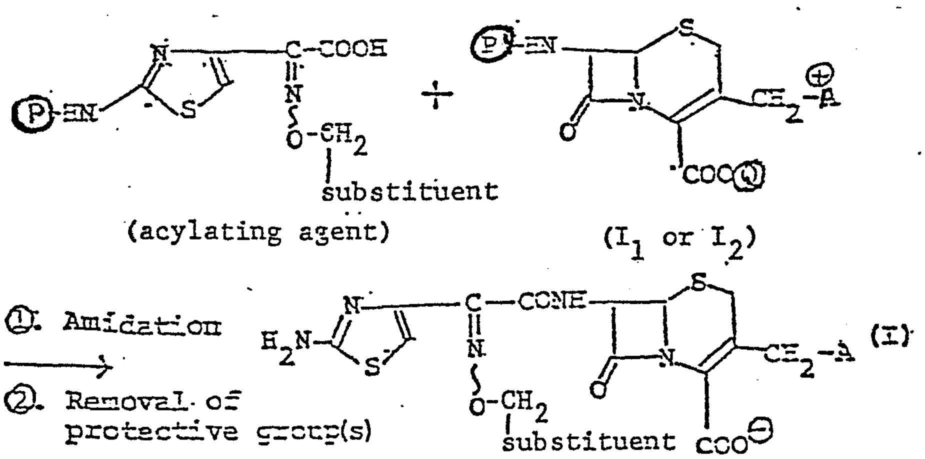


(acylating agent) ($I_1$ or $I_2$)

(I)

In the above formula, P is a hydrogen atom or a protective group for an amino group, P' is a hydrogen atom or a tri-loweralkylsilyl group; and Q is a hydrogen atom or a tri-loweralkylsilyl group: $-A^+$ is as defined above.

Valuable cephalosporin compounds I can thus be produced by reacting sustituted oxyiminothiazolylacetic acid derivatives or reactive derivatives thereof with 7-amino-3-cephem derivatives ($I_1$ and $I_2$) and then, if necessary, releasing any protective group(s).

In this case the protective group for an amino group may be one usually used in the field of peptide chemistry and practical examples are acyl groups such as a formyl group, an acetyl group, a propionyl group, a tert-butoxycarbonyl group, a methoxyacetyl group, a methoxypropionyl group, a benzyloxycarbonyl group, a p-nitrobenzyloxycarbonyl group; tri-loweralkylsilyl groups such as a trimethylsilyl group; and aralkyl groups such as a benzyl group, a benzhydryl group (diphenylmethyl group), a trityl group.

The reaction is usually performed in a solvent under cooling at room temperature or below. Any

solvents which do not take part in the reaction can be used. Examples of the solvent usually used are organic solvents such as dioxane, tetrahydrofuran, ether, acetone, ethyl methyl ketone, chloroform, dichloromethane, dichloroethane, methanol, ethanol, acetonitrile, ethyl acetate, ethyl formate, dimethylformamide, dimethyl sulfoxide; these solvents may be used alone or in appropriate combination.

The acylating agent may be a free carboxylic acid or a reactive derivative thereof. Suitable examples of the compound are mixed acid anhydrides, acid anhydrides, acid halides, active esters, active amides, acid azides. When using the compound in the form of a free carboxylic acid, it is preferred to use a condensing agent such as N,N'-dicyclo-hexylcarbodiimide or N,N'-diethylcarbodiimide.

According to the kind of reactive derivative of carboxylic acid used, it may be preferred for smooth reaction to operate in the presence of a base. Examples of such a base are inorganic bases such as sodium hydrogencarbonate, potassium hydrogencarbonate, sodium carbonate, potassium carbonate; and organic bases such as trimethylamine, triethylamine, dimethylaniline, pyridine.

The invention is further explained and illustrated by the following Reference Examples and Examples. The Reference Examples illustrate producing 7-substituted aminocephalosporin compounds by introducing a substituent at the 7-amino position of the cephalosporin nucleus of the compounds of this invention.

Example 1

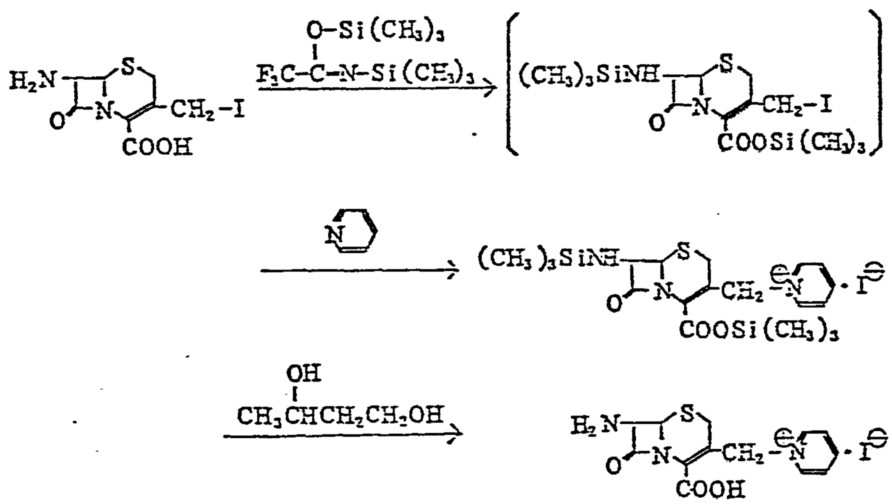

In 10 ml of dichloromethane was suspended 680 mg of 7-amino-3-iodomethyl-3-cephem-4-carboxylic acid and after adding thereto 1.2 ml of O,N-bis(trimethylsilyl)trifluoroacetamide, the mixture was stirred for 30 minutes at

room temperature for form a clear solution. The solution was cooled to 5 to 6°C, and 316 mg of pyridine was added thereto. After stirring the solution for 4 hours at the same temperature, 1.6 ml of 1,3-butanediol was added thereto. Precipitates thus formed were collected by filtration, washed with 30 ml of dichloromethane, and dried to provide 730 mg of 7-amino-3-(1-pyridiniomethyl)-3-cephem-4-carboxylic acid iodide.

NMR(DMSO-$d_6$)

δ 5.09, 5.19 (each 1H, each d, 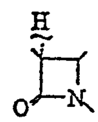 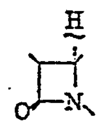

5.58 (2H, s, 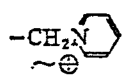 )

8.24 (2H, t, 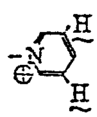 )

8.70 (1H, t, 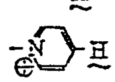 )

9.10 (2H, d, 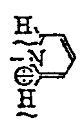 )

Using the compound of Example 1 as a starting material, the following compound was obtained .

Reference Example 1

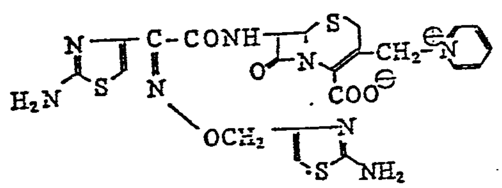

(i) In 10 ml of dichloromethane was suspended 419 mg of 7-amino-3-(1-pyridiniomethyl)-3-cephem-4-carboxylic acid iodide and after adding thereto 0.9 ml of O,N-bis(trimethylsilyl)trifluoroacetamide, the mixture was stirred for 30 minutes to form a clear solution. The solution was cooled to -40°C, and 0.5 ml of pyridine was added thereto. (The solution thus formed is hereafter referred to as "Solution A".)

In 10 ml of dichloromethane was suspended 783 mg of (Z)-α-(2-tritylamino-4-thiazolyl)-α-[(2-tritylamino-4-thiazolyl)methoxyimino]acetic acid and after cooling the suspension to 3 to 4°C, 208 mg of phosphorus pentachloride was added thereto, and the mixture was stirred for 15 minutes at 3-4°C. (The solution thus formed is hereafter referred to as "Solution B".)

Solution B was added dropwise to solution A and the temperature of the reaction mixture was elevated to -10°C over

15 minutes. After adding to the reaction mixture 1 ml of water, 1 ml of tetrahydrofuran and 3 ml of 1N-hydrochloric acid, the mixture was stirred for 10 minutes at -10 - 0°C. The reaction mixture was distilled under reduced pressure to remove dichloromethane and tetrahydrofuran and after adding to the residue obtained 50 ml of water, precipitates (powder) thus formed were collected by filtration, washed with water, and dried to provide 0.91 g of a crude product (a compound having protective groups).

(ii) After adding to the crude product obtained as above 20 ml of trifluoroacetic acid and 6 ml of water under ice-cooling, the mixture was stirred for 1 hour at room temperature. Insoluble materials were removed by filtration, and the filtrate was concentrated under reduced pressure. To the residue was added 20 ml of ether, and the powder thus formed was collected by filtration to provide 0.45 g of a crude product. The crude product was suspended in 50 ml of water and after adding 2 ml of 1N-hydrochloric acid, the formed solution was subjected to column chromatography on Diaion HP-20 and the product was eluted first with water and then with mixtures of water-methanol of successively changing mixing ratio. The fractions containing the desired product were concentrated, and lyophilized to provide 80 mg of (Z)-7-{α-(2-amino-4-thiazolyl)-α-[(2-amino-4-thiazolyl)methoxyimino]acetamido}-3-(1-pyridiniomethyl)-3-cephem-4-carboxylate.

NMR ( DMSO — $d_6$ )

δ ( ppm) : 4.88 ( 2H, 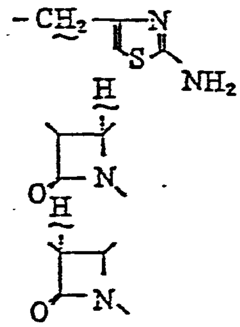 )

5.08 ( 1H, )

5.72 ( 1H, )

6.40, 6.68 (each 1H, 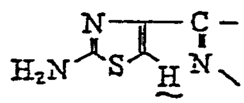

+ 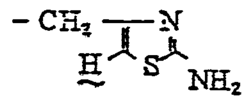 )

8.13 ( 2H, 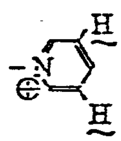 )

8.57 ( 1H, 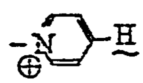 )

9.39 ( 2H, 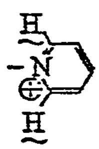 )

Example 2

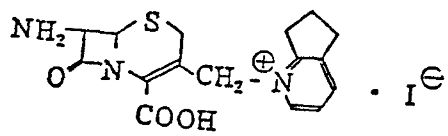

In 50 ml of dichloromethane was suspended 1.7 g of 7-amino-3-iodomethyl-3-cephem-4-carboxylic acid and after adding thereto 2.8 ml of O,N-bis(trimethylsilyl)-trifluoroacetamide, the mixture was stirred for 30 minutes at room temperature to form a clear solution. After adding thereto 654 mg of 2,3-cyclopentenopyridine, the solution was stirred for 4 hours at room temperature to provide trimethylsilyl 7-trimethylsilylamino-3-(2,3-cyclopenteno-1-pyridiniomethyl)-3-cephem-4-carboxylate iodide. After adding thereto first 4 ml of 1,3-butanediol and then adding 150 ml of ether, precipitates thus formed were collected by filtration, washed with 50 ml of ether, and dried to provide 1.8 g of 7-amino-3-(2,3-cyclopenteno-1-pyridiniomethyl)-3-cephem-4-carboxylic acid iodide.

NMR ( DMSO $-$ $d_6$ )

$\delta$ (ppm) 1.90 ∼ 2.40 (2H, m, 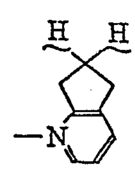 )

2.8～3.9 (6H, m, 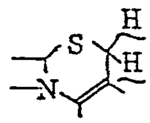 + 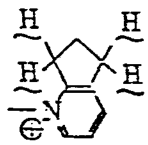 )

5.10, 5.21 (each 1H, each d, 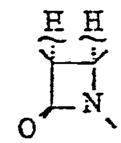 )

5.49 (2H, s, 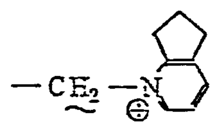 )

7.86 (1H, t, 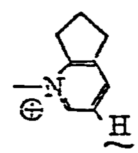 )

8.34 (1H, d, 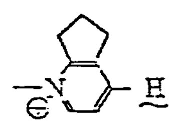 )

8.64 (1H, d, 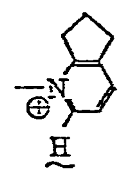 )

Using, as a starting material, trimethylsilyl 7-trimethylsilylamino-3-(2,3-cyclopenteno-1-pyridiniomethyl)-3-cephem-4-carboxylate iodide obtained in the course of Example 2, the following compound was obtained.

Reference Example 2

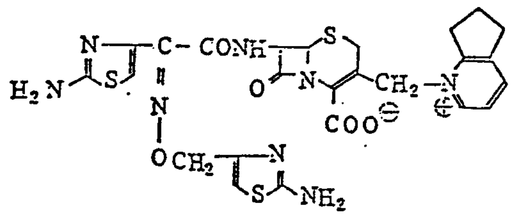

In 25 ml of dichloromethane was suspended 3.92 g of (Z)-α-(2-tritylamino-4-thiazolyl)-α-[(2-tritylamino-4-thiazolyl)methoxyimino]acetic acid and after cooling the suspension to 3 to 4° C and then adding thereto 1.04 g of phosphorus pentachloride, the mixture was stirred for 15 mintues at 3-4 °C. (The solution thus formed is hereafter referred to as "Solution C".)

A solution containing trimethylsilyl 7-trimethylsilylamino-3-(2,3-cyclopenteno-1-pyridiniomethyl)-3-cephem-4-carboxylate iodide obtained in the course of Example 2 was cooled to -50° C, and 2.2 ml of pyridine was added thereto. (The solution thus formed is hereafter referred to as "Solution D").

Solution C was added dropwise to solution D, and the temperature of the reaction mixture was increased to -15°C over 15 minutes. After adding thereto 5 ml of water, 10 ml of tetrahydrofuran, and 10 ml of

1N-hydrochloric acid, the resultant solution was stirred for 10 minutes under ice-cooling. The reaction mixture was distilled under reduced pressure to remove dichloromethane and tetrahydrofuran and after adding to the residue obtained 200 ml of water, precipitates thus formed were collected by filtration. The precipitate obtained was washed with water, and dried to provide 5.5 g of a crude product ( a compound having protective groups). After adding to the crude product 60 ml of trifluoroacetic acid and 12 ml of water under ice-cooling, the mixture was stirred for 1 hour at room temperature. Insoluble materials were removed by filtration, and the filtrate was concentrated under reduced pressure. To the residue was added 150 ml of ether to form a powder, and the powder was collected by filtration to provide 2.7 g of a crude product. The crude product was suspended in 200 ml of water and after adding thereto 4 ml of 1N-hydrochloric acid, the formed solution was subjected to column chromatography on Diaion HP-20 and the product was eluted first with water and then mixtures of water and methanol of successively changing mixing ratio (water:methanol from 10:1 to 10:6). The fractions containing the desired product were concentrated, and lyophilized to provide 275 mg of (Z)-

7-{α-(2-amino-4-thiazolyl)-α-[(2-amino-4-thiazolyl)-methoxyimino]acetamido}-3-(2,3-cyclopenteno-1-pyridinyl)-3-cephem-4-carboxylate.

NMR ( DMSO-$d_6$)

δ : 4.89 (2H, 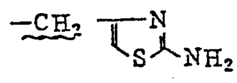 )

5.06 (1H, 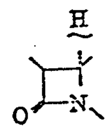 )

5.70 (1H, 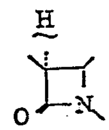 )

6.44, 6.73 (each 1H, 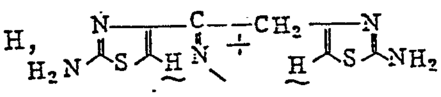 )

7.83 (1H, 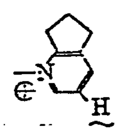 )

8.36 (1H, 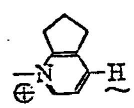 )

9.19 (1H, 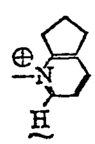 )

Example 3

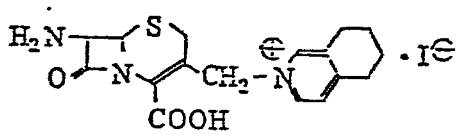

In 50 ml of dichloromethane was suspended 1.7 g of 7-amino-3-iodomethyl-3-cephem-4-carboxylic acid and after adding thereto 2.8 ml of O,N-bis(trimethylsilyl)trifluoroacetamide, the mixture was stirred for 30 minutes at room temperature to form a clear solution. After adding thereto 665 mg of 5,6,7,8-tetrahydroisoquinoline, the solution was stirred for 4 hours at room temperature to provide trimethylsilyl 7-trimethylsilylamino-3-(5,6,7,8-tetrahydro-2-isoquinoliniomethyl)-3-cephem-4-carboxylate iodide. After adding thereto 4 ml of 1,3-butanediol, and then adding 150 ml of ether, precipitates thus formed were collected by filtration, washed with 50 ml of ether, and dried to provide 2 g of 7-amino-3-(5,6,7,8-tetrahydro-2-isoquinoliniomethyl)-3-cephem-4-carboxylic acid iodide.

NMR ( DMSO - $d_6$ )

δ : ppm 1.60~1.96 ( 4H, b, 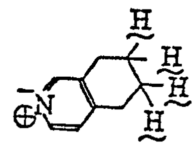 )

2.60~3.12 ( 4H, b, 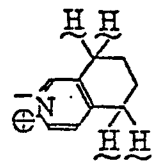 )

4.98~5.08 (each 1H, each d, 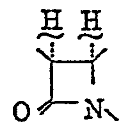 )

5.40 ( 2H, s, 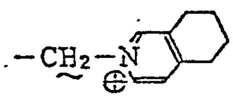 )

7.82 ( 1H, d, 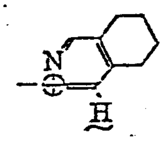 )

8.60 ( 1H, d, 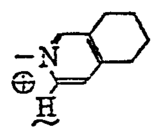 )

8.72 ( 1H, s, 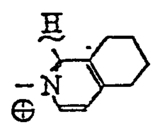 )

Using, as a starting material, trimethylsilyl 7-trimethylsilylamino-3-(5,6,7,8-tetrahydro-2-isoquinoliniomethyl)-3-cephem-4-carboxylate iodide obtained in the course of Example 3, the following compound was produced.

Reference Example 3

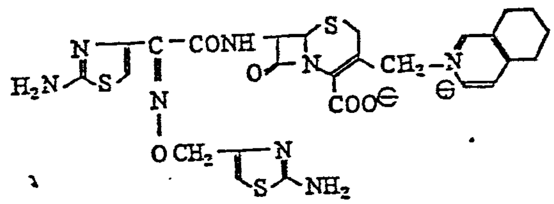

In 25 ml of dichloromethane was suspended 3.92 g of (Z)-α-(2-tritylamino-4-thiazolyl)-α-[(2-tritylamino-4-thiazolyl)methoxyimino]acetic acid and after cooling the suspension to 3 to 4 °C and then adding thereto 1.04 g of phosphorus pentachloride, the mixture was stirred for 15 minutes at 3-4 °C. (The solution thus formed is hereafter referred to as "Solution E".)

A solution containing trimethylsilyl 7-trimethylsilylamino-3-(5,6,7,8-tetrahydro-2-isoquinoliniomethyl)-3-cephem-4-carboxylate iodide obtained in the course of Example 3 was cooled to -50°C, and 2.2 ml of pyridine was added thereto. (The solution thus formed is hereafter referred to as "Solution F").

Solution E was added dropwise to solution F, and the temperature of the reaction mixture was increased to -15°C over 15 minutes. After adding thereto 5 ml of water, 15 ml of 1N-hydrochloric acid, and 10 ml of tetrahydrofuran, the resultant solution was stirred for 10 minutes under ice-cooling. The reaction mixture was distilled under

reduced pressure to remove dichloromethane and tetrahydrofuran and after adding to the residue obtained 200 ml of water, precipitates thus formed were collected by filtration to provide 10 g of a crude product containing water (a compound having protective groups). After adding to the crude product 70 ml of trifluoroacetic acid and 14 ml of water under ice-cooling, the mixture was stirred for 1 hour at room temperature. Insoluble materials were removed by filtration, and the filtrate was concentrated under reduced pressure. To the residue was added 200 ml of ether to form a powder, and the powder was collected by filtration to provide 4 g of a crude product. The crude product was suspended in 300 ml of water and after adding 6 ml of 1N-hydrochloric acid, the formed solution was subjected to column chromatography on Diaion HP-20 and the product was eluted first with water and then mixtures of water and methanol of successively changing mixing ratio (water:methanol from 10:1 to 10:7). The fractions containing the described product were concentrated, and lyophilized to provide 393 mg of (Z)-7-{α-(2-amino-4-thiazolyl)-α-[(2-amino-4-thiazolyl)methoxyimino]acetamido}-3-(5,6,7,8-tetrahydro-2-isoquinoliniomethyl)-3-cephem-4-carboxylate.

NMR ( DMSO $- d_6$ )

1.55～2.00 ( 4 H, 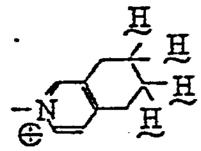 )

2.60～3.20 ( 4 H, 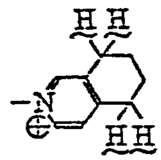 )

4.88 ( 2 H, 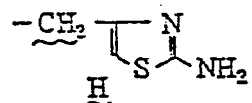 )

5.07 ( 1 H, 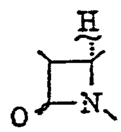 )

5.68 ( 1 H, 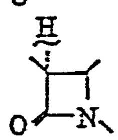 )

6.43, 6.73 (each 1 H, 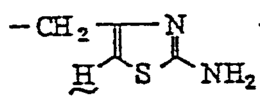 + 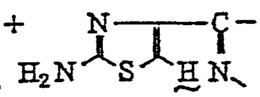 )

7.84 ( 1 H, 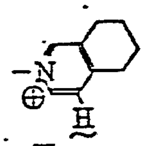 )

9.05 ( 1 H, 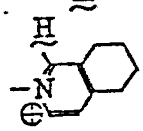 ), 9.09 ( 1 H, 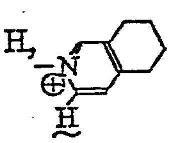 )

CLAIMS:

1. A process for the preparation of 7-amino-3-substituted methyl-3-cephem-4-carboxylic acid or carboxylate represented by the general formula

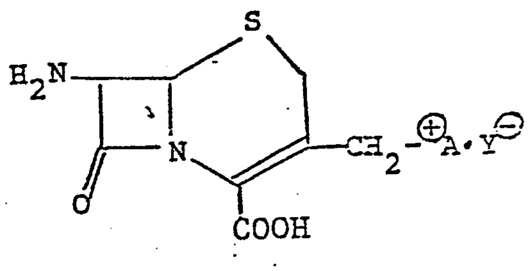

or

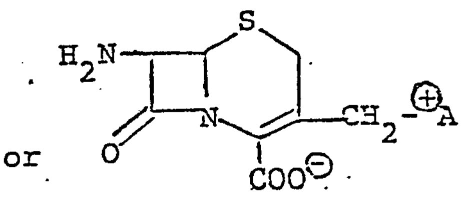

$(I_2)$

which comprises reacting 7-amino-3-halogenomethyl-3-cephem-4-carboxylic acid of the formula (II) or salt thereof

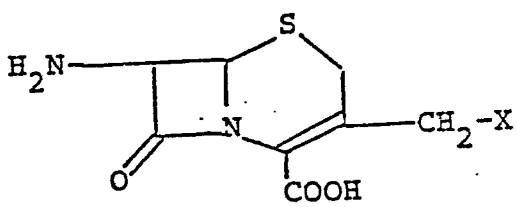

(II)

with (tri-lower-alkylsilyl)trifluoroacetamide (III) selected from

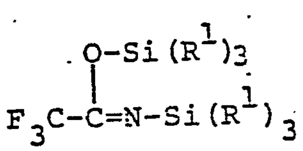

$(III_1)$

and

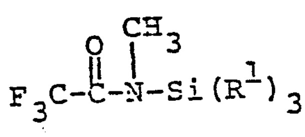

$(III_2)$

and pyridine compound selected from

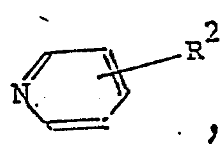

,

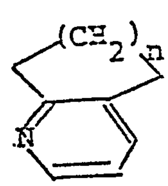

and

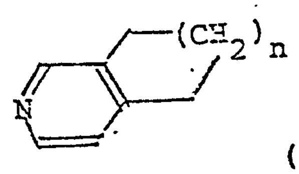

(IV)

and then releasing the protective tri-lower-alkylsilyl groups of the formed compound of the formula

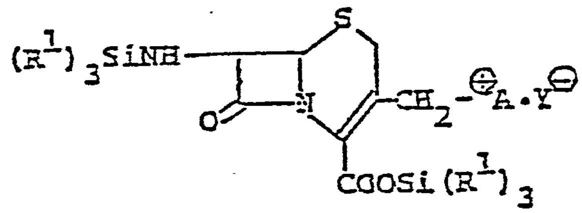

($I_1$)

wherein $-^{\oplus}A$ represents

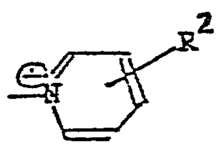

,

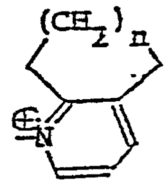

and

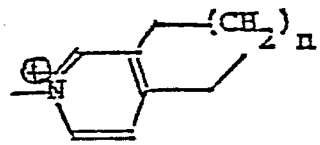

(where $R^2$ is hydrogen or an amino group and n is 1 or 2), $Y^{\ominus}$ is anionic, the $R^1$'s are selected from lower alkyl groups, and X is a halogen.

2. A process for the preparation of tri-loweralkylsilyl derivative represented by the general formula

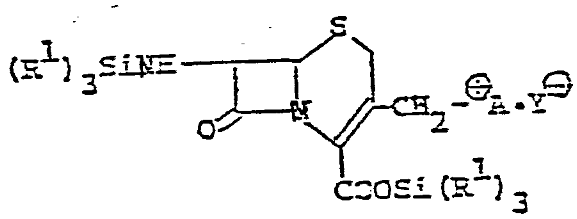

($I_1$)

wherein $-^{\oplus}A$ is selected from

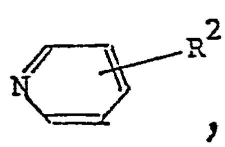

,

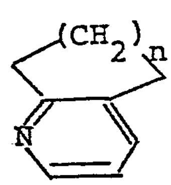

and

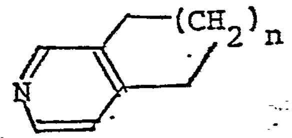

(where $R^2$ is hydrogen or an amino group and n is 1 or 2), $Y^{\ominus}$ is anionic, and the $R^1$'s are selected from lower alkyl groups, which comprises reacting 7-amino-3-halogenomethyl-3-cephem-4-carboxylic acid of the formula (II) or salt thereof

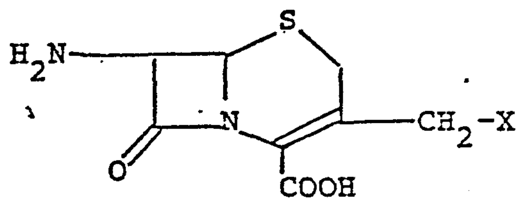

(II)

(where X is halogen) with (tri-loweralkylsilyl)trifluoroacetamide (III) selected from

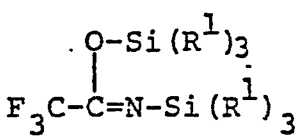

$(III_1)$

and

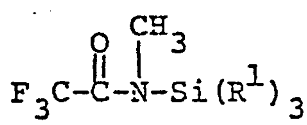

$(III_2)$

and pyridine compound selected from

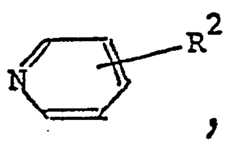

,

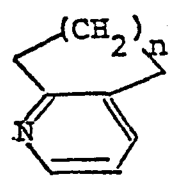

and

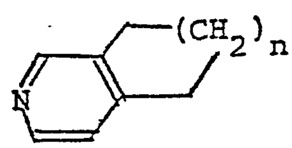

(IV)

3. A process for the preparation of 7-amino-3-substituted methyl-3-cephem-4-carboxylic acid of the formula

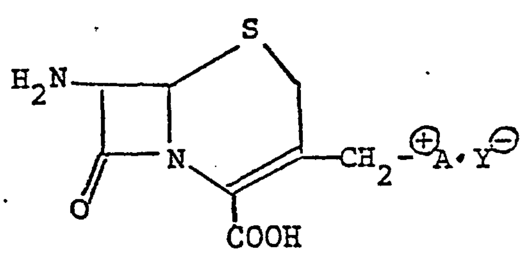

or

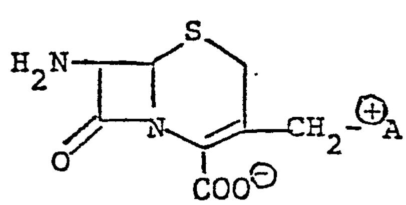

$(I_2)$

which comprises releasing the protective tri-loweralkylsilyl groups of tri-loweralkylsilyl derivative represented by the formula

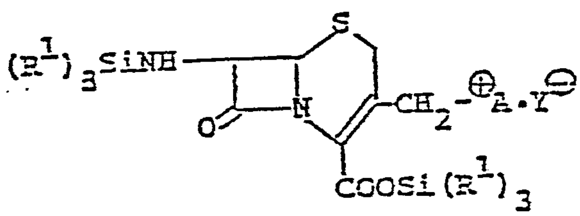 $(I_1)$

wherein $-^{\oplus}A$ is selected from

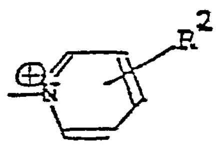 , 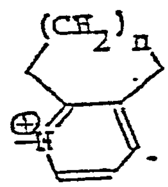 . and 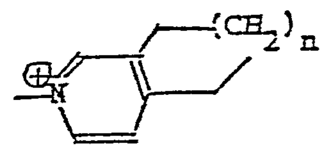

(wherein $R^2$ is hydrogen or an amino group, and n is 1 or 2), $Y^{\ominus}$ is anionic, and the $R^1$'s are selected from lower alkyl groups.

4. A process according to claim 1 or 2 wherein the starting acid or salt and reactant (III) are reacted first, followed by reaction with pyridine compound (IV).

5. A process according to any preceding claim wherein $R^1$ is a methyl group.

6. A process according to any preceding claim wherein Y is inorganic, both X and Y preferably being iodine.

7. Compounds of the formula

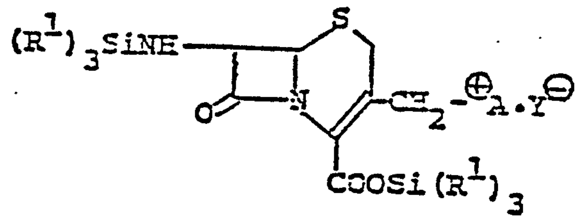

$(I_1)$

wherein $-^{\oplus}A$ is selected from

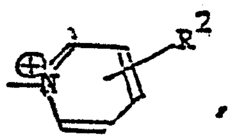

,

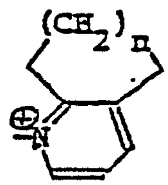

and

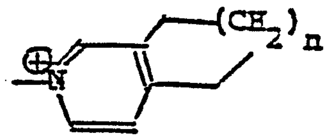

(wherein $R^2$ is hydrogen or an amino group, and n is 1 or 2), $Y^-$ is anionic, the $R^1$'s are selected from lower alkyl groups.

8. Compounds, other than 7-amino-3-pyridiniomethyl-3-cephem-4-carboxylate, of the formulae

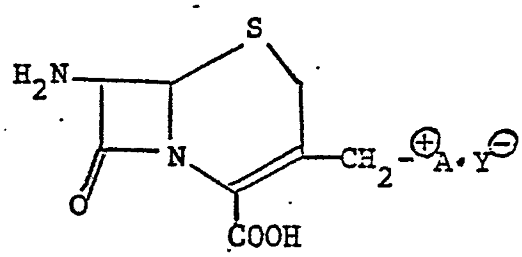

and

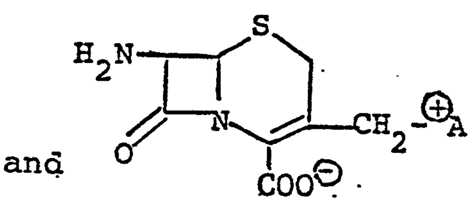

$(I_2)$

wherein $^{\oplus}A$ and $Y^{\ominus}$ are as defined in claim 1.

9. Compounds according to claim 7 wherein $R^1$ is a methyl group.

10. Compounds according to claim 7, 8 or 9 wherein Y is inorganic, preferably halogen, e.g. iodine.